(19) Europäisches Patentamt
European Patent Office
Office européen des brevets

(11) Publication number: **0 002 136**
**B1**

(12) # EUROPEAN PATENT SPECIFICATION

(45) Date of publication of patent specification: **21.10.81**

(51) Int. Cl.³: **C 07 C 121/64,**
C 09 K 3/34, G 09 F 9/35

(21) Application number: **78300649.7**

(22) Date of filing: **21.11.78**

(54) **Substituted benzonitriles, their preparation, liquid crystal compositions containing them and display devices employing such compositions.**

(30) Priority: **21.11.77 JP 139610/77**
**06.06.78 JP 67938/78**

(43) Date of publication of application:
**30.05.79 Bulletin 79/11**

(45) Publication of the grant of the European patent:
**21.10.81 Bulletin 81/42**

(84) Designated Contracting States:
**CH DE FR GB**

(56) References cited:
**FR - A - 2 377 376**

**ANGEWANDTE CHEMIE, International Edition English, 16, nr. 2, 1977 page 100**

(73) Proprietor: **Chisso Corporation**
**6-32, Nakanoshima 3-chome Kitaku**
**Osaka-shi Osaka-fu (JP)**

(72) Inventor: **Sato, Hideo**
**8-10, Nojimacho, Kanazawaku Yokohamashi**
**Kanagawaken (JP)**
Inventor: **Furukawa, Kenji**
**10-1, Otsutomocho, Kanazawaku Yokohamashi**
**Kanagawaken (JP)**
Inventor: **Sugimori, Shigeru**
**10-2, Otsutomocho, Kanazawaku Yokohamashi**
**Kanagawaken (JP)**

(74) Representative: **Ruffles, Graham Keith et al,**
**MARKS & CLERK 57-60 Lincoln's Inn Fields**
**London WC2A 3LS (GB)**

**EP 0 002 136 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Substituted benzonitriles, their preparation, liquid crystal compositions containing them and display devices employing such compositions

The present invention relates to novel compounds which are Np liquid crystal materials, i.e. they form nematic liquid crystals with a positive dielectric anisotropy.

Display equipment which utilizes liquid crystals has recently come into practical use in various fields. Of the display devices available, those which use so-called twisted nematic type liquid crystals cells and wherein the nematic liquid crystals have a positive dielectric anisotropy have come to be the most widely employed. Many demands are placed on the liquid crystal materials and they are rquired to possess a number of characteristics, for example a broad temperature range, good stability, low actuation threshold voltage and short response time, among others. It is difficult for a single compound to fulfil such requirements, and it is thus the present state of the art that admixtures of various compounds are employed in order to obtain the desired properties.

R. Eidenschink *et al* have recently reported (Angw. Chem. *89*, 103 (1977)) that the 4-*trans*-4-alkylcyclohex-yl)benzonitriles expressed by the following formula (II) are superior nematic liquid crystal materials with a low viscosity:

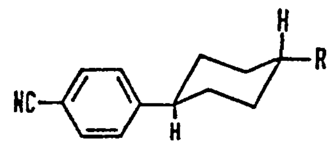

(II)

wherein R represents a straight-chain alkyl group.

The method for preparing such a compound is very complicated because the alkyl group, R, and the cyanophenyl group have to be in the *trans* configuration on the cyclohexane ring in order that a liquid crystal state is obtained. As such, separation of the *trans*-compound from the *cis*-compound is found necessary.

As described in the report by Eidenschink *et al*, a compound (II) is obtained by reacting the corresponding 4-alkylcyclohexanone with phenylmagnesium bromide to synthesize a mixture of *cis*- and *trans*-4-alkylphenylcyclohexanols, separating the *cis*- and *trans*-isomers by chromatography using silica gel, hydrogenating in the presence of a Raney nickel catalyst the resulting *cis*-isomer wherein the R and OH groups are in the *cis* configuration, hydrogenating in the presence of a palladium catalyst the corresponding *trans*-isomer, the two hydrogenations thereby both giving the same *trans*-4-alkyl-1-phenylcyclohexane which is then acetylated and thereafter oxidised using the halo form reaction to give the corresponding carboxylic acid, and the acid is thereafter amidated and dehydrated to give the nitrile. This lengthy sequence can be expressed by the following reaction scheme:

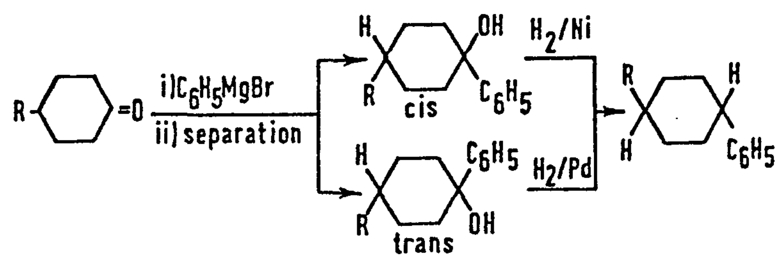


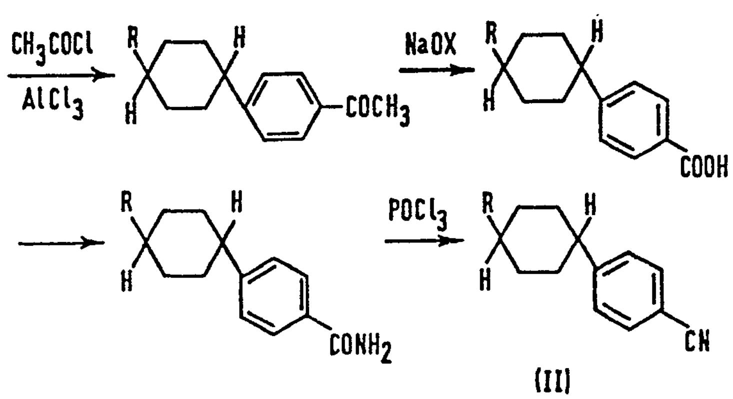


(II)

Apart from the difficulty in their preparation the compounds of formula (II) possess properties

which make them good candidates for use in display devices based on Np liquid crystals.

More recently still, in French Patent Specification No. 2377376 published on 11 August 1978 there have been disclosed corresponding hexahydroterphenyl derivatives such as 4-cyano-4′-(trans-4-alkylkyclohexyl)biphenyls, that is, 4-(*trans*-4-alkylcyclohexyl-1′, 4′-phenylene)benzonitriles. Compared to the compounds disclosed by Eidenschink *et al*, these compounds of French Patent Specification No. 2377376 have a 1,4-phenylene link interposed between the aromatic and aliphatic rings. As with Eidenschink, mention is made of the possible use of the compounds in liquid crystal compositions.

The synthetic route proposed in French Patent Specification No. 2377376 is generally the same as proposed by Eidenschink, but with use of a biphenyl Grignard reagent instead of phenylmagnesium bromide. The difficulty in preparation is thus similar.

The present inventors have found compounds which are analogous to the compounds of formula (II) and to the compounds of the French Patent specification, but which are easier to prepare and which exhibit a stable liquid crystal state and give good results in display devices.

According to the present invention, we provide compounds of formula (I):

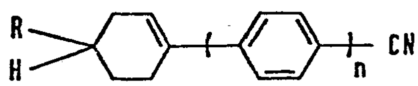

(I)

wherein R represents a straight-chain alkyl group having 1 to 9 carbon atoms and *n* is 1 or 2. When *n* is 1, the compounds are 4-(4-alkyl-1-cyclohexenyl)benzonitriles, otherwise referred to as 1-(*p*-cyanophenyl)-4-alkylhexenes, while when *n* is 2, the compounds are 4-(4″-alkyl-1″-cyclohexenyl-1′,4′-phenylene)benzonitriles, otherwise referred to as 1-(4′-cyano-4-biphenyl)-4-alkylcyclohexenes.

Preferred compounds are those of the general formula

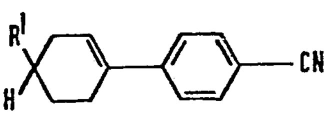

(Ia)

wherein $R^1$ represents a straight-chain alkyl gorup having 3 to 7 carbon atoms, and those of the general formula

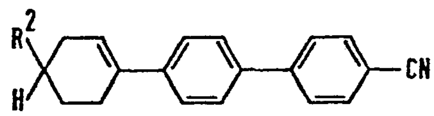

(Ib)

wherein $R^2$ represents a straight-chain alkyl group having 3 to 5 carbon atoms.

The compounds of formula (I) can be prepared by a process which is more simple than that described for compounds of formula (II) by Eidenschink *et al*. This simplicity is founded in part on the discovery that a 4-alkylcyclohexanol substituted at the 1-position with a substituted benzene ring can be dehydrated as it is and without the need to separate out the *cis* and *trans*-isomers.

The present process for preparing a compound of formula (I) comprises three steps. Depending upon whther *n* is 1 or 2 in the desired compound of formula (I), the first step involves *either* reacting a 4-alkylcyclohexanone with 4-bromophenylmagnesium bromide or 4-bromopohenyllithium to prepare a 1-(4-bromophenyl)-4-alkyl cyclohexanol as a mixture of its *cis*- and *trans*-isomers, *or* reacting a 4-alkylcyclohexanone with 4′-bromobiphenyl-4′-yllithium to prepare a 1-(4″-bromobiphenyl-4′-yl)-4-alkylcyclohexanol as a mixture of its *cis*- and *trans*-isomers. The second step then involves converting the mixture of *cis*- and *trans*-isomers into a 1-bromo-4-(4-alkyl-1-cyclohexenyl)benzene (that is, a 1-(*p*-bromophenyl)-4-alkylcyclohexene) or into a 1-(4″-bromobiphenyl-4′-yl)-4-alkylcyclohexene (that is, a 1-(4′-bromo-4-biphenyl-4-alkylcyclohexene), as the case may be, by using a dehydration reaction. In the third step, the bromo substituent is replaced by a cyano group to give the desired compound.

In other words, the present invention provides a method for producing a compound of the general formula

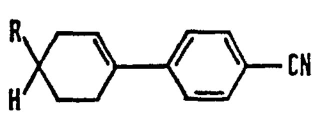

(Ic)

wherein R represents a straight-chain alkyl group having 1 to 9 carbon atoms, which method comprises:

reacting a 4-alkylcyclohexanone (wherein the alkyl group has a straight chain of 1 to 9 carbon atoms) with 4-bromophenylmagnesium bromide or 4-bromophenyllithium to form a mixture of *cis*- and *trans*-1-(4-bromophenyl)-4-alkylcyclohexanols;

subjecting the mixture of *cis-* and *trans-*isomers to dehydration to form a 1-(4-bromophenyl)-4-alkylcyclohexene (ie. a 1-bromo-4-(4-alkyl-1-cyclohexenyl)benzene); and

replacing the bromo group of the compound thus prepared with a cyano group to give the desired compound of formula (I*c*), and it also provides a method for producing a compound of the general formula

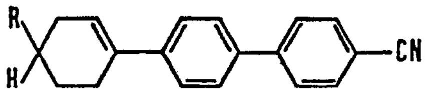

(Id)

wherein R represents a straight-chain alkyl group having 1 to 9 carbon atoms, which method comprises:

reacting a 4-alkylcyclohexanone (wherein the alkyl group has a straight chain of 1 to 9 carbon atoms) with 4-bromo-4′-biphenyllithium to prepare a mixture of *cis-* and *trans-* 1-(4′-bromobiphenyl-4′-yl)-4-alkylcyclohexanols;

subjecting the mixture of *cis-* and *trans-*isomers to dehydration to form a 1-(4′-bromo-4-biphenyl)-4-alkylcyclohexene (ie. a 1-4″-bromobiphenyl-4′-yl)-4-alkylcyclohexene) and

replacing the bromo group of the compound thus prepared with a cyano group to give a compound of formula (I*d*).

In a preferred sequence, the process of the present invention is carried out in the following manner:

Firstly, a 4-alkylcyclohexanone is reacted to prepare either a 1-(4-bromophenyl)-4-alkylcyclohexanol or a 1-(4″-bromobiphenyl-4″-yl)-4-alkylcyclohexanol. For convenience, we refer hereinafter to such a substituted cyclohexanol product as a compound (A). The compounds (A) are prepared as a mixture of *cis* and *trans-*isomers by the reaction of the 4-alkylcyclohexanone with 4-bromophenylmagnesium bromide, 4-bromophenyllithium or 4′-bromobiphenyl-4-yllithium, as may be appropriate.

(In passing, we mention that the 4-alkylcyclohexanones which are required as a starting material in the first step can be obtained by oxidising a mixture of *cis-* and *trans-* forms of the corresponding 4-alkylcyclohexanol which itself can be obtained by hydrogenation of a 4-alkylphenol. The oxidation can be satisfactorily carried out using anhydrous chromic acid, sodium bichromate or the like oxidising agent. Moreover, the 4-bromophenylmagnesium bromide is easily obtained by reacting 1,4-dibromobenzene with an equivalent amount of metallic magnesium, while 4-bromophenyllithium or 4-bromobiphenyl-4′-yllithium is easily obtained by reacting 1,4-dibromobenzene or, as the case may be, 4,4′-dibromobiphenyl with an equivalent amount of butyllithium.)

Secondly, the crude product from the first step and containing a compound (A) is directly subjected as it is to dehydration in the second step in order to prepare the corresponding 1-bromo-4-(4-alkyl-1-cyclohexenyl)benzene or 1-(4″-bromobiphenyl-4′-yl)-4-alkylcyclohexane. These cyclohexanes, which may alternatively be named as 1-(4-bromophenyl)-4-alkylcyclohexenes or 1-(4-bromobiphenyl-4′-yl)-4-alkylcyclohexenes, respectively, are referred to hereinafter as compounds (B). The second step can be carried out with good efficiency by heating a compound (A) either in the absence of a solvent or, more preferably, in the presence of a solvent such as for example toluene or xylene, and by employing an acid catalyst such as potassium hydrogensulphate, oxalic acid, *p*-toluenesulphonic acid or the like catalytic material. In this reaction, the same alkylcyclohexene (B) is formed from either the *cis-*isomer of a compound (A) (i.e. A-*cis*) or from the *trans-*isomer of the compound (A) (i.e. A-*trans*). It is therefore not at all necessary to separate the *cis-* and *trans-*isomers prior to the dehydration. This lack of a need for isomeric separation is a characteristic feature of the present preparative process.

Thirdly, the compound (B) is reacted in a third step with cuprous cyanide in a solvent with a high boiling point, preferably a dipolar aprotic solvent such as N-methyl-2-pyrrolidone, dimethylformamide or the like. This third step then gives the desired 4-(4-alkyl-1-cyclohexenyl)benzonitrile or 4-(4″-alkyl-1″-cyclohexenyl-1′, 4′-phenylene)benzonitrile of the formula (I), that is a 1-(4-cyanophenyl)-4-alkylcyclohexene or a 1-(4″-cyanobiphenyl-4′-yl)-4-alkylcyclohexene.

The preferred process as described above can be represented by the following reaction sequence:

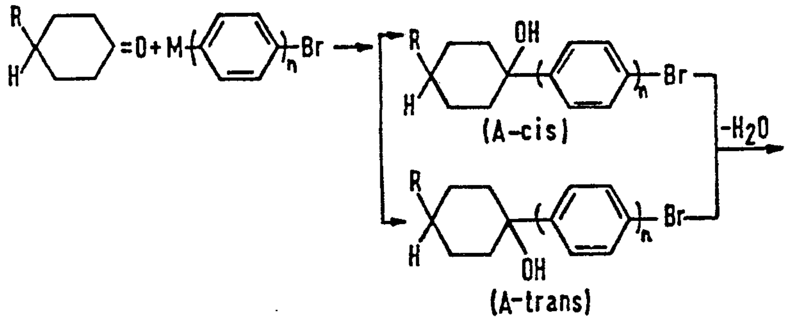


(A-cis)

(A-trans)

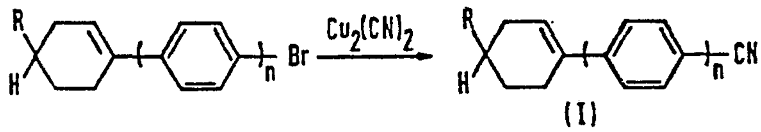


(I)

wherein M represents MgBr and *n* is 1 or M represents Li and *n* is 1 or 2.

In an alternative method for preparing a compound of formula (I), the compound (B) can be prepared by reacting a 4-cyclohexanone with phenylmagnesium bromide or phenyllithium to form a 1-phenyl-4-alkylcyclohexanol or a 1-(biphenyl-4′-yl)-4-alkylcyclohexanol (both these hexanols being a mixture of *cis*- and *trans*-isomers), brominating the substituted cyclohexanol at the desired 4- or 4″-position of the benzene ring and then dehydrating the product to give the compound (B). This method has the disadvantage that the bromination often also occurs at other positions and that purification of the compound (B) is difficult.

It is also conceivable that the compounds (I) could be prepared without the intermediate formation of a compound (B). For example, it is possible to proceed by dehydrating a 1-phenyl-4-alkylcyclohexanol to give a 1-phenyl-4-alkylcyclohexene which is then acylated to give a 1-(4-acetylphenyl)-4-alkylcyclohexene, oxidising this latter compound to give a 1-(4-carboxyphenyl)-4- alkylcyclohexene, and thereafter converting the carboxyl group into a cyano group via formation of a carboxyamide group. However this reaction sequence takes many stages and there is no particular practical advantage to be achieved.

The compounds of formula (I) are liquid crystal materials, and accordingly the invention further provides a nematic liquid crystal composition which contains at least one compound of formula (I).

The compounds of formula (I) of the present invention are characterised by a viscosity which is lower than conventional liquid crystal compounds and which is similar to that of compounds of formula (II) given above and which have a cyclohexane ring. This low viscosity results in the present compounds exhibiting a short response time when they are employed in liquid crystal display devices. The compounds of the present invention are Np liquid crystal materials and it is possible to use a mixture of the present compounds, without other compounds, for a twisted nematic type liquid crystal cell. Moreover the present compounds exhibit good compatibility with other liquid crystal materials such as the liquid crystal compounds with azoxybenzene, alkylbiphenyl or cyanophenyl alkylbenzoate groups, and it is thus possible to use one or more of the present compounds as a mixed type liquid crystal in admixture with other liquid crystal materials.

The present invention therefore provides nematic liquid crystal compositions containing at least one compound of the general formula

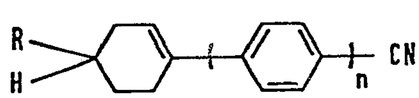

(I)

wherein R represents a straight alkyl group having 1 to 9 carbon atoms and *n* is 1 or 2. Such a composition can contain one or more compounds of formula (I) wherein *n* is 1, and can contain one or more compounds of formula (I) wherein *n* is 2.

The following Table 1 gives details of the properties of the compounds of formula (I) which were prepared as in the following Examples 1 to 12.

TABLE 1

Properties of Compounds of Formula (I)

| n | R | C–N Point (Melting Point) (°C) | N–I Point (Clear Point) (°C) | Example Number |
|---|---|---|---|---|
| 1 | $CH_3$ | 55 (C–I point)** | (−30)*** | 3 |
| 1 | $C_2H_5$ | 22 (C–I point)** | (10)* | 1 |
| 1 | n-$C_3H_7$ | 31 | 44 | 4 |
| 1 | n-$C_4H_9$ | 32.5 | 41.5 | 5 |
| 1 | n-$C_5H_{11}$ | 42 | 57 | 2 |
| 1 | n-$C_6H_{13}$ | 46 | 53.5 | 6 |
| 1 | n-$C_7H_{15}$ | 47.5 | 61 | 7 |
| 1 | n-$C_8H_{17}$ | 32.5 (C–S point)<br>46 (S–N point) | 60 | 8 |
| 2 | $CH_3$ | 157 | 186 | 10 |
| 2 | n-$C_3H_7$ | 110 | 232 | 9 |
| 2 | n-$C_4H_9$ | 113 | 225 | 11 |
| 2 | n-$C_5H_{11}$ | 109 | 225 | 12 |

* : N–I point of monotropic liquid crystal.

** : Since these compounds give monotropic liquid crystals, there is no C–N point.

*** : Values obtained by extrapolation method.

As can be seen from the values for the nematic temperature ranges given in Table 1, the compounds wherein $n = 1$ and R is $C_2H_5$ to $C_7H_{15}$ are particularly suitable as liquid crystal components for liquid crystal display elements, and they can thus be employed as a main component for such an element. Moreover, the compounds wherein $n = 2$ exhibit a liquid crystal state over a relatively wide temperature range and are suitable for formulation of a liquid crystal composition having a broad nematic temperature range by employing them in admixture with compounds (I) wherein $n = 1$. Furthermore, the compounds (I) wherein $n = 2$ can serve the purpose of broadening the nematic temperature range not only of compounds of formula (I) wherein $n = 1$, but also of other nematic liquid crystal materials having a positive dielectric anisotropy. Examples of such other nematic liquid crystal materials include the 4-alkyl-4'-cyanobisphenyls, 1-(4-cyanophenyl)-4'-alkylcyclohexanes, 4-alkylbenzoic acid-4-cyanophenyl esters and the 4-alkylbenzylidene-4′-cyanoanilines. In addition, by adding a compound of formula (I) wherein $n = 2$ to a liquid crystal material which has a negative dielectric anisotropy and is itself unsuitable for a twisted nematic type liquid crystal display cell, it is possible to convert the unsuitable material into a suitable liquid crystal composition having a positive dielectric anistropy.

Preparation methods for the compounds of the present invention will now be described in more detail by way of non-limitative Examples.

Example 1

Preparation of a compound of formula (I) wherein $n = 1$ and $R = C_2H_5$, 4-(4-ethyl-1-cyclohexenyl)-benzonitrile

30.4 g (1.25 mol) of magnesium metal was introduced into a 2 l three-neck flask, dry nitrogen was passed through the flask to ensure the flask was adequately dry, and then 50 ml of anhydrous tetrahydrofuran and a trace of iodine were added. While the resulting mixture was stirred, a solution obtained by dissolving 283.1 g (1.2 mol) of 1,4-dibromobenzene in 600 ml of tetrahydrofuran was added dropwise to the mixture through a dropping funnel over a period of about 1.5 hour, during which time the reaction temperature was maintained at 30 to 35°C by water cooling. After the dropwise addition, the temperature was further maintained at 30 to 35°C for about 1.5 hour in order to complete the formation of 4-bromophenylmagnesium bromide. The flask was then cooled with ice and 128.2 g (1 mol) of 4-ethylcyclohexanone added dropwise over 20 minutes while allowing the temperature to return to room temperature. (The 4-ethylcyclohexanone employed in this case was prepared by oxidizing a commercial grade of 4-ethylcyclohexanol with Jones reagent and had a boiling point of 87 to 89°C/26 mm Hg.) To the above-mentioned reaction product was added a small amount of 6N hydrochloric acid by dropwise addition to acidify the resulting solution, followed by 200 ml of water and 200 ml of hexane. After separation of the layers, the water layer was extracted with 200 ml of hexane and the hexane extracts thus obtained were combined, washed once with water and the solvent distilled off. The resulting residue was a mixture of crude *cis*- and *trans*-isomers of 1-(4-bromophenyl)-4-ethylcyclohexanol (i.e. compound A where $R = C_2H_5$ and $n = 1$.

To this mixture of A-*cis* and A-*trans* compound was added 50 g of potassium hydrogensulphate, and the temperature was gradually raised while maintaining a nitrogen atmosphere and stirring the mix. In this way a dehydration reaction was carried out by heating at 230 to 250°C for 3 hours. After cooling of the product, hexane was added to dissolve out the desired compound. After distilling off of hexane, further distillation was carried out under a reduced pressure to collect a fraction having a boiling point of 126 to 134°C/1 mm Hg (143 g) and which solidified. This product was recrystallized twice from ethanol to give about 110 g of colourless crystals of 1-bromo-4-(4-ethyl-1-cyclohexenyl)benzene, otherwise 1-(p-bromophenyl)-4-ethylcyclohexene i.e. the compound B where $R = C_2H_5$ and $n = 1$, having a melting point of 53 to 54°C.

100 g of this B compound was heated together with 41 g of cuprous cyanide in 300 ml of N-methyl-2-pyrrolidone under reflux for 4 hours, followed by distillation off of about 200 ml of N-methyl-2-pyrrolidone under atmospheric pressure. The resulting residue was treated with an aqueous solution of ferric chloride at 50°C. Hexane-extraction, acid-washing, alkali-washing, and recrystallization from ethanol were then carried out to give 52 g of colourless crystals of the desired 4-(4-ethyl-1-cyclohexenyl)benzonitrile (i.e. 1-(*p*-cyanophenyl)-4-ethylcyclohexene) with yield: 65%. This product was a monotropic liquid crystal material having a C—I point (melting point) of 22°C and a N—I point of 10°C. Further the elemental analysis values of this product showed good agreement with the theoretical values, as will be apparent from the following figures for $C_{15}H_{17}N$:

| | Analytical Value (%) | Theoretical Value (%) |
|---|---|---|
| C | 85.1 | 85.26 |
| H | 8.2 | 8.11 |
| N | 6.5 | 6.63 |

Moreover the NMR and infrared absorption spectra of the product were entirely consistent with the product being the compound of formula (I) where $R = C_2H_5$ and $n = 1$.

Example 2

Preparation of a compound of formula (I) wherein $R = C_5H_{11}$ and $n = 1$, 4(4-pentyl-1-cyclohexenyl)benzonitrile

236 g (1 mol) of 1,4-dibromobenzene and 1 l of dry toluene were introduced into a three-neck flask under a dry nitrogen atmosphere and dissolution effected with stirring at 50°C. To the resulting solution was dropwise added 525 ml of a hexane solution of 1.67N commercial butyllithium (0.88 mol), the addition being over a period of 30 minutes. After the addition, the temperature was maintained at 50°C for a further 30 minutes and then lowered down to 25°C. To the solution was then dropwise added over a period of 30 minutes a solution obtained by dissolving 135 g (0.8 mol) of 4-pentylcyclohexanone in 125 ml of toluene. The stirring was thereafter continued for 30 minutes at 30°C, and then 250 ml of 6N hydrochloric acid was added dropwise while maintaining the temperature at 30°C or lower, thus completing the first step.

(The 4-pentylcyclohexanone was obtained by hydrogenating 4-pentylphenol in the presence of a Raney nickel catalyst to form 4-pentylcyclohexanol (b.p.: 122 to 130°C/10 mm Hg), and by then oxidizing this material with Jones reagent (b.p. of product: 130 to 134°C/22 mm Hg)).

The resulting organic layer was separated, 25 g of potassium hydrogen sulphate was added thereto, and after hexane was distilled off with stirring, the resulting residue was concentrated until the liquid temperature reached 110°C, thereby completing the dehydration reaction of the second step.

The resulting concentrate was distilled under a reduced pressure to give 160 g of a fraction at 160 to 170°C/1 mm Hg which was 1-bromo-4-(4-pentyl-1-cyclohexenyl)benzene, i.e. 1-(*p*-bromophenyl)-4-*n*-pentylcyclohexene, a compound B where $R = n\text{-}C_5H_{11}$ and $n = 1$, in a yield of 62.4%. The product was then recrystallized twice from ethanol to give crystals having a melting point of 67 to 68°C.

The cyanogenation reaction, the third step, was carried out in the same manner as in Example 1 to give the desired product (b.p.: 153 to 161°C/0.5 mm Hg, yield: about 90%), which was then recrystallized from methanol to give colourless crystals. This was a Np liquid crystal material and had a C—N point of 42 to 42.4°C and a N—I point of 56.7°C. Elemental analysis of the product accorded well with the theoretical values for $C_{18}C_{23}N$:

| | Analytical Value (%) | Theoretical Value (%) |
|---|---|---|
| C | 85.1 | 85.32 |
| H | 9.2 | 9.15 |
| N | 5.5 | 5.5 |

The NMR and infrared absorption spectra of this product were consistent with the product being the desired compound.

Examples 3 to 8

3: 4-(4-methyl-1-cyclohexenyl)benzonitrile
4: 4-(4-propyl-1-cyclohexenyl)benzonitrile
5: 4-(4-butyl-1-cyclohexenyl)benzonitrile
6: 4-(4-hexyl-1-cyclohexenyl)benzonitrile
7: 4-(4-heptyl-1-cyclohexenyl)benzonitrile
8: 4-(4-octyl-1-cyclohexenyl)benzonitrile

These Examples were carried out in the same manner as in Example 1 or Example 2 except that the 4-alkylcyclohexanol employed as a starting material for each of Examples 3 to 8 had an alkyl group corresponding to that of the desired product which respectively were products of formula (I) where $n = 1$ and $R = CH_3$, $n\text{-}C_3H_7$, $n\text{-}C_4H_9$, $n\text{-}C_6H_{13}$, $n\text{-}C_7H_{15}$ or $n\text{-}C_8H_{17}$. The properties of the products as liquid crystals are shown together with those of other compounds in Table 1 given above.

Example 9

Preparation of compound of formula (I) wherein $R = n\text{-}C_3H_7$ and $n = 2$, 4-(4''-propyl-1''-cyclohexenyl-1',4'-phenylene)benzonitrile

312 g (1 mol) of 4,4'-dibromobiphenyl and 1.51 of dry toluene were introduced into a 3 l three-necked flask with a dry nitrogen atmosphere and at 55°C to give a solution. To this solution was added dropwise over a period of about 30 minutes 660 ml of a hexane solution of 1.67N commercial butyllithium (1.1 mol); during the addition the solution temperature was maintained at 50 to 55°C. After cooling of the resulting mixture down to 25°C, 140 g (1 mol) of 4-propylcyclohexanone was

added dropwise over 30 minutes while maintaining the temperature at 25—30°C. After the addition, 50 ml of water was added dropwise over 30 minutes with stirring at 30°C, followed by dropwise adding of 200 ml of 6N hydrochloric acid while maintaining the temperature at 30°C or lower.

(The 4-propylcyclohexanone was prepared as follows: a commercial 4-propylphenol was hydrogenated in the presence of ethanol, sodium ethoxide and Raney nickel catalyst, under a hydrogen pressure of 100 atm, at 150°C to form 4-propylcyclohexanol, which was then oxidized with Jones reagent ($CrO_3/H_2SO_4/H_2O$) in acetone to obtain 4-*n*-propylcyclohexanone having a b.p. of 110—113°C/26 mm Hg.)

The resulting liquid layers were separated and the lower water layer was discarded. To the upper organic layer, a solution consisting mainly of 1-(4''-bromobiphenyl-4'-yl)-4-propylcyclohexanol in *cis*- and *trans*-forms, was added 20 g of potassium hydrogensulphate ($KHSO_4$) followed by distilling off of the solvent by heating and concentration until the solution temperature reached 110°C. During this period, the required dehydration reaction proceeded. The resulting solution while hot was transferred to an Erlenmeyer flask to precipitate the compound (B) where R = 2n-$C_3H_7$ and $n = 2$, containing a small amount of 4,4'-dibromobiphenyl. The precipitate was then filtered off and collected. A fraction boiling at 220 to 250°C/3 mm Hg was obtained by vacuum distillation. Recrystallization from toluene gave 206g of the compound (B) where R = *n*-$C_3H_7$ and $n = 2$, yield 58% based on raw material. This product was pale yellow crystals, and melted at 165°C to give a smectic liquid crystal; at 235°C it formed an isotropic liquid.

195.4 g (0.55 mol) of this B compound, i.e., 1-(4''-bromobiphenyl-4'-yl)-4-propylcyclohexene, 57.5 g (0.32 mol) of cuprous cyanide, $Cu_2(CN)_2$, and 550 ml of N-methyl-2-pyrrolidone were heated together in a three-neck flask under reflux with stirring for 5 hours. 500 ml of toluene was added thereto, the mixture was cooled, and a solution consisting of 65 g of ferric chloride, 16 ml of conc hydrochloric acid and 750 ml of water was added and the mixture stirred for 30 minutes at 60 to 70°C. The resulting layers were separated and the organic layer washed with dilute hydrochloric acid, then with dilute aqueous sodium hydroxide, and further with water. After a small amount of solid matter had been eliminated by filtration, toluene was distilled off, and vacuum-distillation was carried out to obtain in crude form 127 g of a fraction boiling at 200 to 210°C/1 mm Hg which was 4-(4''-propyl-1''-cyclohexenyl-1',4'-phenylene)benzonitrile, a compound of formula (I) where R = *n*-$C_3H_7$ and $n = 2$, yield: 76% based on the compound (B). The product was then dissolved in toluene, the resulting solution passed through an alumina layer to effect chromatographic purification, the toluene solution concentrated and the product recrystallized to give 105 g of the purified compound of formula (I), 4(4''-propyl-1''-cyclohexenyl-1'4'-phenylene)benzonitrile, i.e. 1-(4-cyano-4-biphenylyl)-4-*n*-propylcyclohexene. This product had a melting point (C—N point) of 110°C and a clear point (N—I point) of 232°C, and elemental analysis of this product accorded well with the calculated values for $C_{22}H_{23}N$:

| | Analytical Values (%) | Calculated Values (%) |
|---|---|---|
| C | 87.5 | 87.66 |
| N | 7.7 | 7.69 |

Further, the infrared absorption spectra of this product were consistent with that for the desired compound.

Examples 10 to 12

10: 4-(4''-methyl-1''-cyclohexenyl-1',4'-phenylene)benzonitrile
11: 4-(4''-butyl-1''-cyclohexenyl-1',4'-phenylene)benzonitrile
12: 4-(4''-pentyl-1''-cyclohexenyl-1',4'-phenylene)benzonitrile

Example 9 was repeated except that the 4-*n*-propylcyclohexanone of Example 9 was replaced by 4—$CH_3$—, 4-*n*-$C_4H_9$— or 4-*n*-$C_5H_{11}$-cyclohexanone, to obtain compounds of formula (I) wherein $n = 2$ and R = $CH_3$, *n*-$C_4H_9$ or *n*-$C_5H_{11}$, respectively. Their properties as liquid crystals are shown together with those of other compounds in Table 1.

The present invention will now be further described by way of Examples which relate to the use of the compounds of formula (I) in liquid crystal cells.

Example 13

A 1:1 by weight mixture of 4-(4-pentyl-1-cyclohexenyl)benzonitrile, i.e. 1-(4-cyanophenyl)-4-*n*-pentylcyclohexene, as obtained in Example 2 and 4-(4-heptyl-1-cyclohexenyl)benzonitrile, i.e. 1-(4-cyanophenyl)-4-*n*-heptyl-cyclohexene, as obtained in Example 7 was prepared and found to behave as a nematic liquid crystal with a positive dielectric anistropy, i.e. it was a Np liquid crystal material, at

25°C and formed an isotropic liquid at 59°C. The viscosity of this mixture was 36cp at 25°C, which is a much lower value than those of conventional liquid crystal compositions. The mixture was sealed in a twisted nematic type liquid crystal cell having a thickness of 10 μm; its actuation threshold voltage was measured to be 1.8V, and its saturation voltage was 3.0V.

Example 14

A liquid crystal mixture consisting of 213 parts of 4-(4-ethyl-1-cyclohexenyl)benzonitrile, i.e. 1-(4-cyanophenyl)-4-*n*-ethylcyclohexene, as obtained in Example 1, 38.5 parts of 4-(4-pentyl-1-cyclohexenyl)benzonitrile, i.e. 1-(4-cyanophenyl)-4-*n*-pentylcyclohexene as obtained in Example 2 and 38.5 parts of 4-(4-heptyl-1-cyclohexenyl)benzonitrile i.e. 1-(4-cyanophenyl)-4-*n*-heptylcyclohexene, as obtained in Example 7 was prepared. The mixture had a liquid crystal temperature range of −15 to 48°C, and its viscosity at 25°C was as low as a value as 25cp. A twisted nematic type liquid crystal cell of 8.5 μm thick was constructed employing the mixture. The actuation threshold voltage was measured to be 1.5V, and the actuation saturation voltage was 2.2V. Furthermore, the response times were short, with a rise time of 70 msec and a decay time of 50 msec, both at 25°C, while even at 0°C, the rise time and the decay time were 500 msec and 250 msec, respectively. Accordingly when this liquid crystal mixture is employed, it is possible even at 0°C to effect an on-off actuation for display of seconds. The short response time is due to the low viscosity of this liquid crystal mixture.

Example 15

A liquid crystal mixture consisting of 17 parts of 4-(4-pentyl-1-cyclohexenyl)benzonitrile, i.e. 1-(4-cyanophenyl)-4-*n*-pentylcyclohexene, as obtained in Examnple 2, 17 parts of 4-(4-heptyl-1-cyclohexenyl)benzonitrile i.e. 1-(4-cyanophenyl)-4-*n*-heptylcyclohexene, as obtained in Example 7, 24 parts of 4-methoxy-4′-ethylazoxybenzene and 42 parts of 4-methoxy-4′-butylazoxybenzene was prepared. It did not crystallize even at −20°C and maintained a liquid crystal state up to 68°C. A twisted nematic type liquid crystal cell having a thickness of 10 μm was filled with the liquid crystal mixture and had an actuation threshold voltage of 2.5V and an actuation saturation voltage of 3.5V. This liquid crystal mixture can thus be suitably employed as a liquid crystal material for multiplex driving.

Example 16

A liquid composition having the following formulation was prepared:

4-(4-propyl-1-cyclohexenyl)benzonitrile, i.e.
1-(4-cyanophenyl)-4-propylcyclohexene (a compound of formula (I) wherein $R = n\text{-}C_3H_7$, $n = 1$) 0.38 mol

1-(4-pentyl-1-cyclohexenyl)benzonitrile, i.e.
1-(4-cyanophenyl)-4-pentylcyclohexene (a compound of formula (I) wherein $R = n\text{-}C_5H_{11}$, $n = 1$) 0.29 mol

1-(4-hexyl-1-cyclohexenyl)benzonitrile, i.e.
1-(4-cyanophenyl)-4-hexylcyclohexene (a compound of formula (I) wherein $R = n\text{-}C_6H_{13}$, $n = 1$) 0.16 mol

1-(4-heptyl-1-cyclohexenyl)benzonitrile, i.e.
1-(4-cyanophenyl)-4)-heptylcyclohexene (a compound of formula (I) wherein $R = n\text{-}C_7H_{15}$, $n = 1$ 0.12 mol

4-(4″-propyl-1″-cyclohexenyl-1′,4′-phenylene)benzonitrile, i.e.
1-(4′-cyanobiphenyl-4-yl)-4-*n*-propylcyclohexene (a compound of formula (I) wherein $R = n\text{-}C_3H_7$, $n = 2$) 0.043 mol

This liquid crystal composition had a clear point of 60°C, and even when it was preserved at −10°C over a period of several months, no crystals precipitated. Furthermore, the viscosity of this liquid crystal composition was 35cp at 25°C. The composition was sealed into a twisted nematic type liquid crystal cell having a thickness of 10 μm. The actuation threshold voltage and actuation saturation voltage were found to be 1.5V and 2.2V, respectively. As for the response times, the rise time and decay time were 90 msec and 70 msec, respectively (measured at 3V, 32 Hz and 25°C).

Example 17

A liquid crystal composition having the following formulation was prepared:

| | |
|---|---|
| 4-(4-propyl-1-cyclohexenyl)benzonitrile, i.e. 1-(4-cyanophenyl)-4-*n*-propylcyclohexene | 0.45 mol |
| 4-(4-pentyl-1-cyclohexenyl)benzonitrile, i.e. 1-(4-cyanophenyl)-4-*n*-pentylcyclohexene | 0.34 mol |
| 4-(4-heptyl-1-cyclohexenyl)benzonitrile, i.e. 1-(4-cyanophenyl)-4-*n*-heptylcyclohexene | 0.16 mol |
| 4-(4″-propyl-1″-cyclohexenyl-1′,4′-phenylene)benzonitrile, i.e. 1-(4′-cyanobiphenyl-4-yl)-4-*n*-propylcyclohexene | 0.053 mol |

The resulting composition had a clear point of 61°C, and the lower limit of its nematic temperature range was −4°C. Furthermore its viscosity was 35cp at 25°C. The composition was sealed into a twisted nematic type liquid crystal cell having a thickness of 9.5 μm. The actuation threshold voltage and actuation saturation voltage were measured to be 1.5 V and 2.2V, respectively. As for the response times, the rise time and the decay time were 90 msec and 75 msec, respectively (measured at 25°C, 3V and 32 Hz).

Example 18

A liquid crystal composition having the following formulation was prepared:

| | |
|---|---|
| 1-(4-propyl-1-cyclohexenyl)benzonitrile, i.e. 1-(4-cyanophenyl)-4-*n*-propylcyclohexene | 0.32 mol |
| 1-(4-butyl-1-cyclohexenyl)benzonitrile, i.e. 1-(4-cyanophenyl)-4-*n*-butylcyclohexene | 0.26 mol |
| 1-(4-pentyl-1-cyclohexenyl)benzonitrile, i.e. 1-(4-cyanophenyl)-4-*n*-pentylcyclohexene | 0.23 mol |
| 1-(4-heptyl-1-cyclohexenyl)benzonitrile, i.e. 1-(4-cyanophenyl)-4-*n*-heptylcyclohexene | 0.094 mol |
| 4-(4″-methyl-1″-cyclohexenyl-1′,4′-phenylene)benzonitrile, i.e. 1-(4′-cyanobiphenyl-4-yl)-4-methylcyclohexene | 0.029 mol |
| 4-(4″-propyl-1″-cyclohexenyl-1′,4′-phenylene)benzonitrile, i.e. 1-(4′-cyanobiphenyl-4-yl)-4-*n*-propylcyclohexene | 0.034 mol |
| 4-(4″-butyl-1″-cyclohexenyl-1′,4′-phenylene)benzonitrile, i.e. 1-(4′-cyanobiphenyl-4-yl)-4-*n*-butylcyclohexene | 0.029 mol |

The resulting composition had a clear point of 78°C, and even when it was preserved at −10°C over a period of several months, no crystals precipitated. This composition was sealed in a twisted nematic type liquid crystal cell having a thickness of 10 μm. The actuation characteristics were measured at 25°C and showed an actuation threshold voltage of 1.4V and an actuation saturation voltage of 2.2V. With regard to the response times, the rise response time and decay response time were 110 msec and 90 msec, respectively (measured at 25°C, 3V and 32 Hz).

Example 19

A liquid crystal mixture consisting of
15 parts by weight of 4,4′-di-*n*-butylazoxybenzene,
23 parts by weight of 4-methoxy-4′-ethylazoxybenzene, and
47 parts by weight of 4-methoxy-4′-*n*-butylazoxybenzene

has a negative dielectric anistropy and hence cannot be employed for a twisted nematic type liquid crystal cell.

On the other hand a liquid crystal composition obtained by further adding thereto
8 parts of 4-(4″-propyl-1″-cyclohexenyl-1′,4′-phenylene)benzonitrile (1-(4′-cyano-4-biphenylyl)-4-*n*-propylcyclohexene) and
8 parts of 4-(4″-pentyl-1″-cyclohexenyl-1′,4′-phenylene)benzonitrile (1-(4-cyanobiphenyl-4′-yl)-4-*n*-pentylcyclohexene

both being compounds of formula (I) of the present invention, has a positive dielectric anisotropy, does not crystallize even at −15°C, and maintains its nematic state up to 93°C. Employing this liquid crystal composition, a twisted nematic type liquid crystal cell having a clearance thickness of 9.5 μm was constructed and its actuation characteristics were measured. The actuation threshold voltage was 2.5V and the actuation saturation voltage was 3.4V. This liquid crystal composition can be suitably employed as a liquid crystal material for multiplex driving.

Example 20

A liquid crystal composition consisting of
4-*n*-pentyl-4′-cyanobiphenyl (11 parts),
4-*n*-heptyl-4′-cyanobiphenyl (27 parts),
4-*n*-octyloxy-4′-cyanobiphenyl (16 parts) and
4-(4″-propyl-1″-cyclohexenyl-1′,4′-phenylene)benzonitrile,

i.e. 1-(4-cyanobiphenyl-4′-yl)-4-*n*-propylcyclohexene (11 parts) has a nematic liquid crystal temperature range of −15°C to +62°C and a viscosity at 25°C of 45 cp. This composition was sealed into a twisted nematic type liquid crystal cell having a thickness of 10 μm and its actuation threshold voltage and its actuation saturation voltage were measured and found to be 1.5V and 2.2V, respectively. The rise response time and decay response time were 90 msec and 90 msec, respectively (measured at 3V, 32 Hz, and 25°C).

Example 21

A liquid crystal compoosition consisting of
4-*n*-propylcyclohexyl-4′-cyanobenzene (34 parts),
4-*n*-pentylcyclohexyl-4′-cyanobenzene (34 parts),
4-*n*-heptylcyclohexyl-4′-cyanobenzene (22 parts) and
4-(4″-propyl-1″-cyclohexenyl-1′,4′-phenylene)benzonitrile.

i.e. 1-(4-cyanobiphenyl-4′-yl)-4-*n*-propylcyclohexene (10 parts) has a nematic liquid crystal temperature range of −10°C to +68°C, and its viscosity at 25°C is 25 cp. This liquid crystal composition was sealed in a twisted nematic type liquid crystal cell having a thickness of 10 μm and the actuation threshold voltage and actuation saturation voltage of this cell were measured. The voltages were 1.5V and 2.4V, respectively. Furthermore the rise response time and decay response time were 110 msec and 60 msec, respectively (measured at 3V, 32 Hz and 25°C).

**Claims**

1. A compound of the general formula

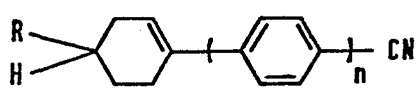

(I)

wherein R represents a straight-chain alkyl group having 1 to 9 carbon atoms and *n* is 1 or 2.

2. A compound of the general formula

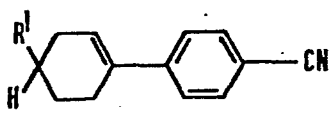

(Ia)

wherein $R^1$ represents a straight-chain alkyl group having 3 to 7 carbon atoms.

3. A compound of the general formula

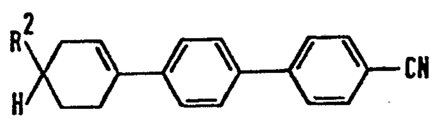

(Ib)

wherein $R^2$ represents a straight-chain alkyl group having 3 to 5 carbon atoms.

4. A method for producing a compound of the general formula

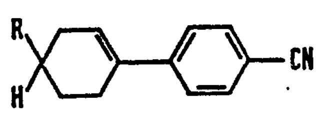

(Ic)

wherein R represents a straight-chain alkyl group having 1 to 9 carbon atoms, which method comprises:

reacting a 4-alkylcyclohexanone (wherein the alkyl group has a straight chain of 1 to 9 carbon atoms) with 4-bromophenylmagnesium bromide or 4-bromophenyllithium to form a mixture of *cis*- and *trans*- 1-(4-bromophenyl)-4-alkylcyclohexanols;

subjecting the mixture of *cis*- and *trans*-isomers to dehydration to form a 1-(4-bromophenyl)-4-alkylcyclohexene (i.e. a 1-bromo-4-(4-alkyl-1-cyclohexenyl)benzene; and

replacing the bromo group of the compound thus prepared with a cyano group to give the desired compound of formula (I*c*).

5. A method for producing a compound of the general formula

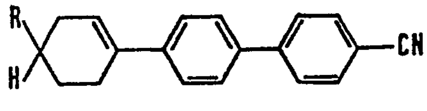

(Id)

wherein R represents a straight-chain alkyl group having 1 to 9 carbon atoms, which method comprises:

reacting a 4-alkylcyclohexanone (wherein the alkyl group has a straight chain of 1 to 9 carbon atoms) with 4-bromo-4′-biphenylyllithium to prepare a mixture of *cis*- and *trans*- 1-(4″-bromobiphenyl-4′-yl)-4-alkylcyclohexanols;

subjecting the mixture of *cis*- and *trans*-isomers to dehydration to form a 1-(4′-bromo-4-biphenylyl)-4-alkylcyclohexene (i.e. a 1-(4″-bromobiphenyl-4′-yl)-4-alkylcyclohexene) and

replacing the bromo group of the compound thus prepared with a cyano group to give a compound of formula (I*d*).

6. A nematic liquid crystal composition containing at least one compound of the general formula

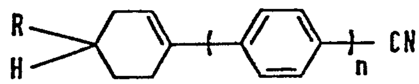

(I)

wherein R represents a straight alkyl group having 1 to 9 carbon atoms and *n* is 1 or 2.

7. A Np liquid crystal display device which employs a composition as claimed in Claim 6.

**Revendications**

1. Composé répondant à la formule générale:

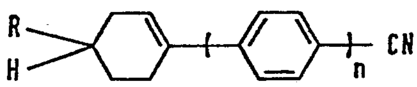

(I)

dans laquelle R représente un groupe alkyle linéaire en $C_1$ à $C_9$ et n est égal à 1 ou 2.

2. Composé réponsant à la formule générale:

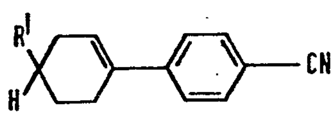

(Ia)

dans laquelle $R^1$ représente un groupe alkyle linéaire en $C_3$ à $C_7$.

3. Composé répondant à la formule générale:

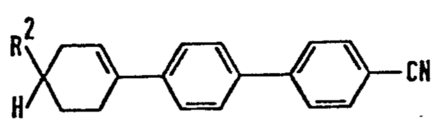

(Ib)

dans laquelle $R^2$ représente un groupe alkyl linéaire en $C_3$ à $C_5$.

4. Procédé de préparation d'un composé répondant à la formule générale:

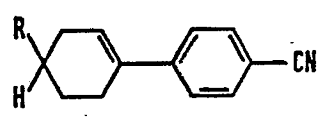

(Ic)

dans laquelle R représente un groupe alkyle linéaire en $C_1$ à $C_9$, caractérisé en ce que:

on fait réagir une 4-alkylcyclohexanone (dans laquelle le groupe alkyle a une chaîne linéaire en $C_1$ à $C_9$) avec le bromure de 4-bromophénylmagnésium ou le 4-bromophényllithium pour former un mélange de 1-(4-bromo-phényl)-4-alcylcyclohenanols *cis* et *trans*;

on soumet le mélange d'isomères cis et trans à une deshydratation pour former le 1-(4-bromophényl)-4-alkylcyclohexène (c'est-à-dire le 1-bromo-4-(4-alkyl-1-cyclohexényl-benzène); et

on remplace le groupe bromo du composé ainsi préparé par un groupe cyano pour obtenir le composé répondant à la formule (Ic) désiré.

5. Procédé de préparation d'un composé répondant à la formule générale:

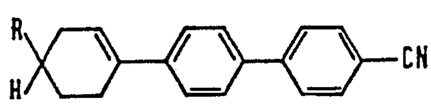

(Id)

dans laquelle R représente un groupe alkyle linéaire en $C_1$ à $C_9$, caractérisé en ce que:

on fait réagir une 4-alkylcyclohexanone (dans laquelle le groupe alkyle a une chaîne linéaire en $C_1$ à $C_9$) avec le 4-bromo-4'-biphénylyllithium pour préparer un mélange de 1-(4''-bromobiphényl-4'-yl)-4-alkylcyclohexanols *cis* et *trans*;

on soumet le mélange d'isomères *cis* et *trans* à une déshydratation pour former un 1-(4'-bromo-4-biphénylyl)-4-alkylcyclohexène (c'est-à-dire un 1-(4''-bromobiphényl-4'-yl)-4-alkylcyclohexène) et

on remplace le groupe bromo du composé ainsi préparé par un groupe cyano pour obtenir le composé répondant à la formule (Id).

6. Composition de cristaux liquides nématiques contenant au moins un composé répondant à la formule générale:

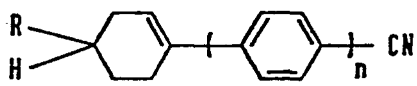

(I)

dans laquelle R représente un groupe alkyle linéaire en $C_1$ à $C_9$ et n est égale à 1 ou 2.

7. Dispositif d'affichage à cristaux liquides Np utilisant une composition suivant la revendication 6.

**Patentansprüche**

1. Verbindung der allgemeinen Formel

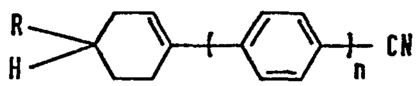

(I)

wobei R eine geradkettige Alkylgruppe mit 1 bis 9 Kohlenstoffatomen darstellt und n 1 oder 2 ist.

2. Verbindung der allgemeinen Formel

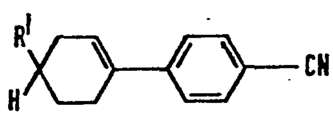

(Ia)

wobei $R^1$ eine geradkettige Alkylgruppe mit 3 bis 7 Kohlenstoffatomen darstellt.

3. Verbindung der allgemeinen Formel

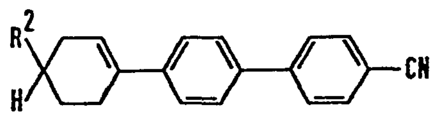

(Ib)

wobei $R^2$ eine geradkettige Alkylgruppe mit 3 bis 5 Kohlenstoffatomen darstellt.

4. Verfahren zur Herstellung einer Verbindung der allgemeinen Formel

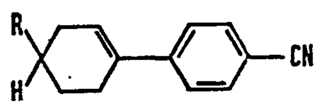

(Ic)

wobei R eine geradkettige Alkylgruppe mit 1 bis 9 Kohlenstoffatom darstellt, bei welchem man 4-Alkylcyclohexanon (in welchem die Alkylgruppe einen geraden Kettenaufbau mit 1 bis 9 Kohlenstoffatomen hat) mit 4-Bromphenylmagnesiumbromid oder 4-Bromphenyllithium reagieren läßt, um eine Mischung von Cis- und Trans-1-(4-Bromphenyl)-4-alkylcyclohexanol herzustellen, bei dem man die Mischung des Cis- und Trans-Isomers einer Dehydrierung unterwirft, um 1-(4-Bromphenyl)-4-alkylcyclohexen (d.h. ein 1-Brom-4-(4-alkyl-1-cyclohexenyl)Benzol) zu bilden, und bei dem man die Bromgruppe der auf diese Weise hergestellten Verbindung durch eine Cyanogruppe ersetzt, um die gewünschte Verbindung der Formel (Ic) zu erhalten.

5. Verfahren zur Herstellung einer Verbindung der allgemeinen Formel

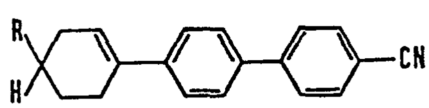

(Id)

wobei R eine geradkettige Alkylgruppe mit 1 bis 9 Kohlenstoffatomen darstellt, bei welchem man ein 4-Alkylcyclohexanon (bei welchem die Alkylgruppe geradkettig aufgebaut ist und 1 bis 9 Kohlenstoffatome enthält) mit 4-Brom-4'-biphenylyllithium reagieren läßt, um eine Mischung aus Cis- und Trans-1-(4''-brombiphenyl-4'-yl)-4-alkylcyclohexanol herzustellen, bei welchem man die Mischung von Cis- und Trans-Isomeren einer Dehydrierung unterwirft, um ein 1-(4'-Brom-4-biphenylyl)-4-alkylcyclohexen (d.h. ein 1-(4''-Brombiphenyl-4'-yl)-alkylcyclohexen) herzustellen, und bei dem man die Bromgruppe der auf diese Weise hergestellten Verbindung durch eine Cyanogruppe ersetzt, um die Verbindung der Formel (Id) zu erhalten.

6. Eine nematische Flüssigkristallverbindung mit mindestens einem Bestandteil der allgemeinen Formel

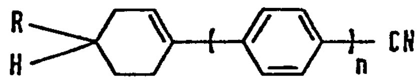

(I)

wobei R eine geradkettige Alkylgruppe mit 1 bis 9 Kohlenstoffatomen darstellt und n 1 oder 2 ist.

7. Eine Np-Flüssigkristallanzeigevorrichtung, welche eine Verbindung benutzt, wie sie in Anspruch 6 beschrieben ist.